**Europäisches Patentamt**

**European Patent Office**

**Office européen des brevets**

(19)

(11) Veröffentlichungsnummer: **0 080 623**

**A2**

(12) # EUROPÄISCHE PATENTANMELDUNG

(21) Anmeldenummer: **82110379.3**

(22) Anmeldetag: **10.11.82**

(51) Int. Cl.³: **A 61 B 3/12**

(30) Priorität: **01.12.81 DE 3147524**

(43) Veröffentlichungstag der Anmeldung:
**08.06.83 Patentblatt 83/23**

(84) Benannte Vertragsstaaten:
**DE FR GB**

(71) Anmelder: **Heine Optotechnik GmbH & Co. KG**
**Kientalstrasse 7**
**D-8036 Herrsching(DE)**

(72) Erfinder: **Heine, Helmut, Dipl. Phys.**
**Gachenaustrasse 10**
**D-8036 Herrsching(DE)**

(72) Erfinder: **Rosenbusch, Helmut**
**Wichernstrasse 8**
**D-8120 Weilheim(DE)**

(72) Erfinder: **Schmidt, Otto Heinz, Dipl. Ing.**
**Schützenweg 36**
**D-8036 Herrsching(DE)**

(74) Vertreter: **Ebbinghaus, Dieter et al,**
**v. FÜNER, EBBINGHAUS, FINCK Patentanwälte**
**European Patent Attorneys Mariahilfplatz 2 & 3**
**D-8000 München 90(DE)**

(54) **Handophthalmoskop.**

(57) Das erfindungsgemäße Handophthalmoskop weist ein allseits geschlossenes Gehäuse 1 auf, in dem die optischen Komponenten 7, 8, 9, 13, 14 völlig staubgeschützt untergebracht sind. Die notwendigen Schaltvorgänge für das Einschalten von Korrekturlinsen 15, 16 Leuchtfeldblenden, Farbfiltern, usw. in den Beleuchtungs- und/oder Beobachtungsstrahlengang 2, 3 werden von elektromagnetischen Antrieben 17, 18, 19, 20 ausgeführt, die durch außen am Gehäuse 1 an ergonomisch günstigster Stelle angeordnete Betätigungelemente 21, 22, 23 bedient werden. Die Anzeige der Brechwerte von im Beobachtungsstrahlengang eingeschalteten Korrekturlinsen 15, 16 sowie daraus ableitbarer Informationen erfolgt auf opto-elektronischem Wege.

Fig. 1

EP 0 080 623 A2

v. FÜNER        EBBINGHAUS

PATENTANWÄLTE        EUROPEAN PATENT ATTORNEYS

MARIAHILFPLATZ 2 & 3, MÜNCHEN 90

POSTADRESSE: POSTFACH 95 01 60, D-8000 MÜNCHEN 95

0080623

— 1 —

Heine Optotechnik GmbH & Co.KG            EPA-30334
                                          10.November 1982


## Handophthalmoskop


Die Erfindung bezieht sich auf ein Handophthalmoskop, in dessen Beleuchtungssystem eine längs ihrer optischen Achse verschiebbare Abbildungsoptik vorgesehen ist, und/oder Blenden, Test- und Meßmarken eines Blendenträgers und/oder die Filter eines Filterträgers einschaltbar sind, und in dessen Beobachtungsstrahlengang die Linsen wenigstens eines Linsenträgers einschaltbar sind, und mit von der Außenseite des Gehäuses zugänglichen bzw. sichtbaren Betätigungs- bzw. Anzeigeelementen.

Moderne Ophthalmoskope enthalten eine Vielzahl von Korrekturlinsen, Leuchtfeldblenden, Meß- und Testmarken und Farbfilter, deren Einstellung dem Untersuchungszweck entsprechend ständig vom Untersucher verändert werden muß. Die Träger dieser optischen Elemente, die auch Bedienungselemente sind, ragen aus dem Ophthalmoskopgehäuse heraus, so daß sie mit den Fingern der haltenden Hand während der Untersuchung direkt bedient werden können. Der Brechwert der jeweils ins Beobachtungssystem eingeschalteten Linse kann vergrößert und beleuchtet durch ein Fenster im Gehäuse abgelesen werden. Ebenso läßt sich die Einstellung der Träger für die Meß- und Testmarken sowie Farbfilter am Träger selbst oder durch Fenster ablesen.

EPA-30334                         - 2 -                         0080623


Ophthalmoskope in dieser konventionellen Bauweise haben folgende Nachteile:

Durch die Gehäuseöffnungen, aus denen die Bedienungselemente
herausragen, kann Staub eindringen und sich auf den optischen
Bauteilen absetzen, mit der Folge, daß die Funktion des Ophthalmoskops durch verringerte Lichtleistung oder durch die optische Abbildung von Staubteilchen im Leuchtfeld auf dem Augenhintergrund beeinträchtigt wird.

Die Anordnung der optischen Elemente und damit die Lage der Bedienungselemente richtet sich weniger nach ergonomischen, als
nach optischen Gesichtspunkten. Dadurch ist es fast immer unvermeidlich, daß der Untersucher der Bedienung des Instruments
unverhältnismäßig viel Aufmerksamkeit widmen oder sogar die
Untersuchung unterbrechen muß.

Der Erfindung liegt daher die Aufgabe zugrunde, ein Handophthalmoskop zu schaffen, das eine ergonomisch möglichst günstige
Anordnung der Betätigungselemente und einen sicheren Staubschutz der optischen Bauteile ermöglicht.

Diese Aufgabe wird erfindungsgemäß dadurch gelöst, daß innerhalb des Gehäuses für die Abbildungsoptik und/oder den Blendenträger und/oder den Filterträger und/oder den Linsenträger je
ein mit dem jeweiligen Träger gekoppelter elektromagnetischer
oder elektromotorischer Antrieb angeordnet ist, daß die Betätigungselemente an der Außenseite des Gehäuses angeordnet sind,
ohne die Gehäusewandung zu durchbrechen, und daß den Trägern
Stellungsfühler zugeordnet sind, die über Signalwandler an die
zugehörigen Anzeigeelemente angeschlossen sind.

Aus der US-PS 4 200 362 ist bereits bekannt, in einem stationären Ophthalmoskop einen Elektromotor zur Verstellung einer Blendenanordnung vorzusehen. Es werden dabei jedoch keine optischen Elemente, sondern der Ablauf der fotografischen Aufnahme anhand von Abschaltungsblenden gesteuert.

Da bei dem erfindungsgemäßen Handophthalsmoskop die Träger zu
ihrer Betätigung nicht durch Öffnungen im Gehäuse hindurch
nach außen zu ragen brauchen, läßt sich das Gehäuse allseitig
verschließen und so ein absolut sicherer Staubschutz erzielen.
Dabei können die Betätigungselemente unabhängig von der örtlichen Lage der zugehörigen Träger an beliebiger Stelle des Gehäuses, und somit ergonomisch optimal angeordnet werden. Die
Stellung der einzelnen Träger und die mit Hilfe der Linsenträger eingestellte Refraktion des zu untersuchenden Auges läßt
sich mittels der Stellungsfühler auf elektro-optischem Weg,
beispielsweise mittels einer Flüssigkristallanzeige nach außen
darstellen.

Bei einer bevorzugten Weiterbildung des erfindungsgemäßen
Handophthalmoskops ist zwischen die Betätigungselemente und
die Antriebe eine elektronische Steuereinheit geschaltet, die
eine zentrale Steuerung sämtlicher Träger ermöglicht.

Die begrenzte Baugröße eines Handophthalmoskops erlaubt es
nicht, die für eine lückenlose Abdeckung eines großen Korrekturbereichs mit möglichst geringer Abstufung der Brechwerte
erforderliche Anzahl von Linsen auf einem einzigen Linsenträger unterzubringen. Man verwendet deshalb eine oder mehrere
Zusatzlinsen, die auf einem zweiten Träger eingesetzt sind,
und die mit den Linsen im ersten Träger kombiniert werden
können. Eine Anzeige des Gesamtbrechwertes ist dabei nur mit
aufwendigen Konstruktionen realisierbar. Statt dessen werden
daher die Einzelbrechwerte angezeigt und die Berechnung
des Gesamtbrechwertes bleibt dem Untersucher überlassen.

Außerdem ist häufig die Ermittlung der interessierenden wahren Patientenrefraktion auf Grund des für ein scharfes Fundusbild eingeschalteten Korrekturwertes nur mit Hilfe von Formeln oder Tabellen möglich. Falls der Untersucher fehlsichtig ist und ohne Brille ophthalmoskopiert, muß er dabei auch stets seine eigene Refraktion berücksichtigen. Ferner werden bei der Bestimmung von Niveau-Unterschieden auf dem Patientenfundus bekanntlich zwei aufeinanderfolgende Einstellungen der Korrekturwerte vorgenommen. Die Faustformel: 3 Dioptrien Differenz entsprechen 1 mm Niveau-Unterschied ergibt bei hohen Fehlsichtigkeiten des Patienten Fehler bis zu 50 %.

Diesen Nachteilen hilft eine bevorzugte Weiterbildung des erfindungsgemäßen Handophthalmoskops ab, bei der der bzw. die Stellungsfühler des Beobachtungssystems an einen Rechner angeschlossen sind, wobei auf der Außenseite des Gehäuses eine Daten- und eine Befehlseingabe für den Rechner angeordnet sind.

Durch entsprechende Programmierung des Rechners läßt sich die wahre Patientenrefraktion unter Berücksichtigung individueller Parameter, also z.B. des Untersuchungsabstandes zwischen Patientenauge und Ophthalmoskop und der Refraktion des Untersuchers, mühelos berechnen und anzeigen. Auch lassen sich Niveauunterschiede auf der Netzhaut des Patientenauges exakt ausmessen und anzeigen.

Wird ein Ausgang des Rechners an den Antrieb der Abbildungsoptik angeschlossen, läßt sich eine stets scharfe Abbildung von Meß- und Testmarken auf dem Patientenfundus auch bei Verwendung mehrerer Linsenträger erreichen, die sonst mit mechanisch vernünftigem Aufwand nur möglich ist, wenn das Ophthalmoskop nur einen Linsenträger enthält. Außerdem läßt sich bei dieser Ausführungsform die Abbildungsoptik entsprechend der Refraktion des Patientenauges entlang ihrer optischen Achse so verschieben, daß sich ein konstanter Abbildungsmaßstab auf dem Patientenfundus ergibt.

Da beim Ophthalmoskopieren von Untersuchung zu Untersuchung die einzelnen Träger jeweils in ihre Null- oder in ihre neutrale Stellung zurückgeführt werden müssen, ergibt sich eine wesentliche Vereinfachung der Handhabung durch einen Nullstellungs-Befehlsgeber, durch den diese Rückstellung über die Antriebe automatisch und gleichzeitig erfolgt. Dabei ist es zweckmäßig, eine optische und/oder akustische Anzeige vorzusehen, die dem Benutzer die vollzogene Rückstellung der Träger signalisiert.

Die Erfindung wird im folgenden anhand der in der Zeichnung dargestellten Ausführungsbeispiele näher erläutert: Es zeigen

Fig. 1 in vergrößerter Darstellung die Seitenansicht eines Handophthalmoskops bei aufgebrochenem Gehäuse,
Fig. 2 in vergrößertem Maßstab die Vorderansicht (von der Untersucher-Seite) des Handophthalmoskops bei aufgebrochenem Gehäuse,
Fig. 3, 4 und 5 die Seitenansicht von rechts, die Vorderansicht von der Untersucher-Seite bzw. die Seitenansicht von links eines Handophthalmoskops, und
Fig. 6 ein Blockschaltbild der einzelnen Elemente eines Handophthalmoskops.

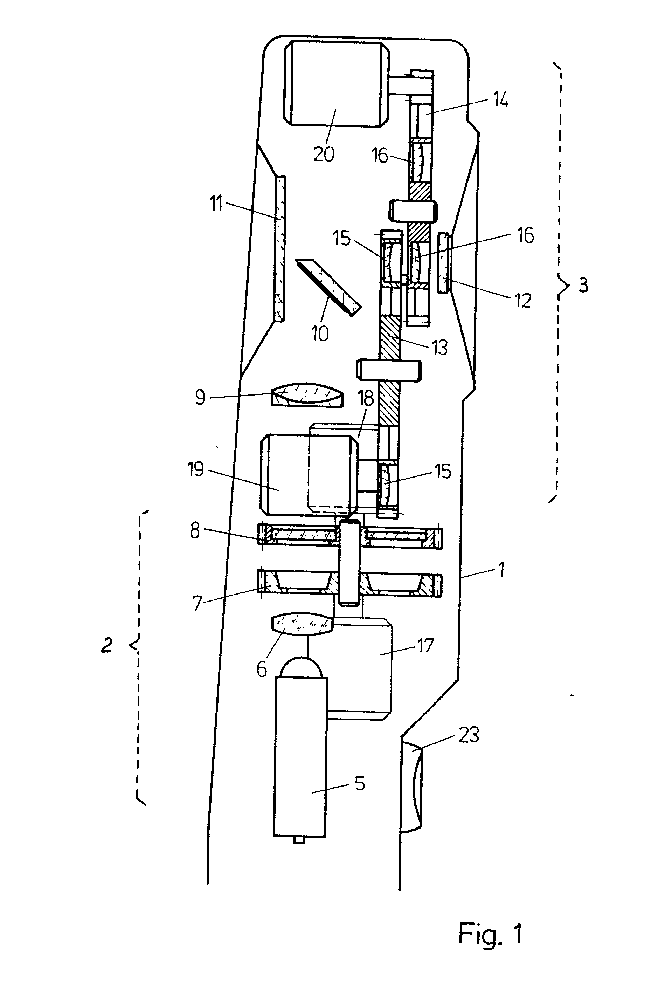

Gemäß Fig. 1 sind im allseits geschlossenen Gehäuse 1 eines Handophthalmoskops dessen Beleuchtungssystem 2 und Beobachtungssystem 3 untergebracht.

Das Beleuchtungssystem 2 umfaßt eine Lichtquelle 5, die als Glühbirne ausgebildet ist. Der Lichtquelle 5 folgen ein Kondensor 6, ein Blendenträger 7, ein Filterträger 8, deren Blenden, Test- und Meßmarken sowie Filter jeweils in den Strahlengang des Beleuchtungssystems 2 einschaltbar sind, eine gegebenenfalls aus mehreren, längs ihrer optischen Achse relativ zueinander verschiebbaren Teilen bestehende Abbildungsoptik 9 und ein Umlenkspiegel 10, der den Beleuchtungsstrahl durch ein Austrittsfenster 11 zum Patientenauge reflektiert.

Das Beobachtungssystem 3 umfaßt ein gegebenenfalls als Linse ausgebildetes Blickfenster 12 sowie ein zur Grobeinstellung dienendes erstes und zur Feineinstellung dienendes zweites Linsenrad 13 bzw. 14, deren Linsen 15 bzw. 16 selektiv in den Beobachtungsstrahlengang einschaltbar sind. Die Brechwerte der Linsen 15 und 16 sind jeweils untereinander um größere und kleinere Stufen, beispielsweise 5 Dioptrien bzw. 1 Dioptrie, derart abgestuft, daß der in der Praxis notwendige Bereich umfaßt wird. Enthält beispielsweise das Grobeinstellungs-Linsenrad 13 zwölf von +20 bis -35 um jeweils 5 Dioptrien und das Feineinstellungs-Linsenrad 14 von 0 bis +4 Dioptrien um jeweils 1 Dioptrie abgestufte Linsen, so läßt sich der Bereich von -35 bis +24 Dioptrien in Schritten von jeweils 1 Dioptrie überstreichen.

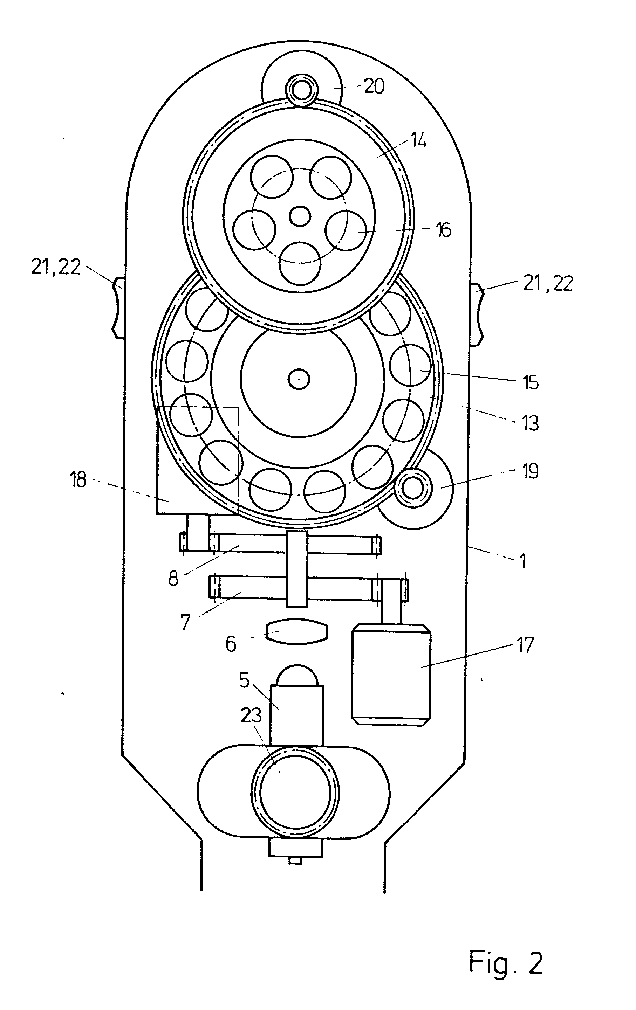

Gemäß Fig. 1 und 2 ist dem Blendenträger 7, dem Filterträger 8 und den beiden Linsenrädern 13 und 14 je ein Antrieb 17, 18, 19 bzw. 20 zugeordnet. Die Antriebe können aus Hubmagneten, Schrittmotoren oder dergleichen bestehen.

Als Bedienungselemente sind auf jeder Seite des Gehäuses 1 (Fig. 2) auf- und abbewegliche Schiebetaster 21 zur Betätigung des Grobeinstellungs-Linsenrades 13 angeordnet, hinter denen (in Fig. 2 nicht sichtbar) Schiebetaster 22 zur Betätigung des Feineinstellungs-Linsenrades 14 angeordnet sind. Da auf beiden Seiten des Gehäuses 1 identische Schiebetaster 21, 22 angeordnet sind, läßt sich das Handophthalmoskop für Rechts- und Linkshänder gleichermaßen gut bedienen. Ein weiterer Schiebetaster 23 dient durch (gemäß der Darstellung der Fig. 2) Verschieben nach links zur Verstellung des Blendeträgers 7 und durch Verschieben nach rechts zum Verstellen des Filterträgers 8. Durch Drücken auf den Schiebeschalter 23 lassen sich sämtliche Träger in ihre Null- oder Ausgangsstellung zurückstellen.

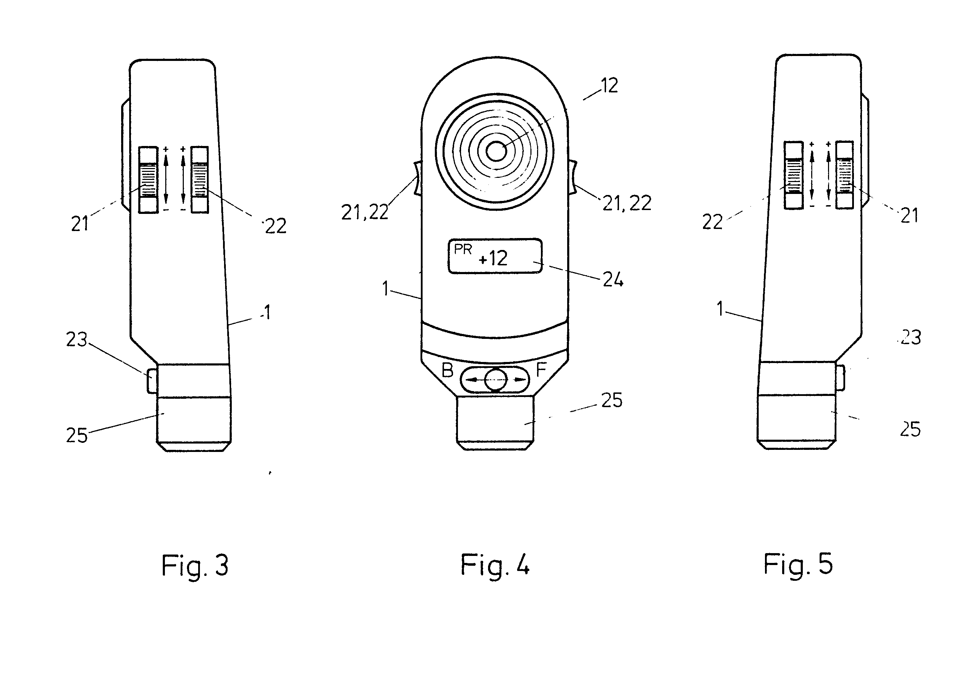

Fig. 3, 4 und 5 zeigen das Handophthalmoskop bei geschlossenem Gehäuse 1. Hinter einem Sichtfenster ist eine Anzeige 24, beispielsweise eine LC-Anzeige, angeordnet, an der sich der durch die Linsenräder 13 und 14 eingestellte Refraktionswert ablesen läßt. Falls die Einstellung des Blendenträgers 7 und des Filterträges 8 nicht durch weitere Sichtfenster angezeigt wird, läßt sich deren Stellung auch über eine mit der Anzeige 24 kombinierte Anzeige darstellen.

Natürlich lassen sich hier auch alle anderen Ergebnisse irgendwelcher Berechnungen darstellen, z.B. die wahre Patientenrefraktion unter Berücksichtigung der Untersuchungsparameter, die Größe von Niveauunterschieden auf dem Augenhintergrund, der Teil-Abstand von auf dem Augenhintergrund projizierten Meßmarken, usw..Zudem ist die Anzeige 24 als Kontrolle für die eingegebenen Untersuchungsparameter zu verwenden.

Der Fuß 25 des Gehäuses ist als Anschluß für die Stromversorgung ausgebildet.

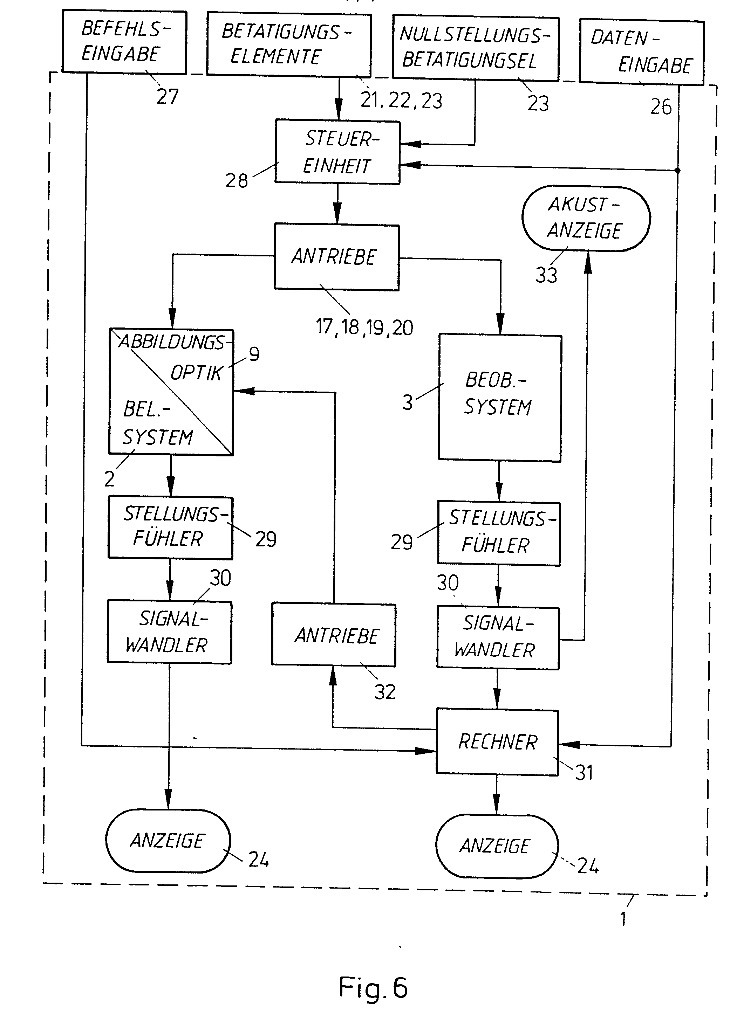

Gemäß der Darstellung der Fig. 6 sind die Betätigungselemente 21, 22,23 sowie eine Dateneingabe 26 und eine Befehlseingabe 27 für einen Rechner auf der Außenseite des Gehäuses 1

angeordnet. An die Ausgänge der Betätigungselemente 21, 22 23 ist eine elektronische Steuereinheit 28 angeschlossen, mit deren Ausgang wiederum die Antriebe 17, 18, 19, 20 verbunden sind. Die Antriebe 17, 18 wirken auf den Blendenträger 7 und den Filterträger 8. Die Antriebe 19, 20 wirken auf die beiden Linsenräder 13, 14 des Beobachtungssytems 3.

Sowohl das Beleuchtungssystem 2 als auch das Beobachtungssystem 3 enthalten je einen Stellungsfühler 29, der die Stellung der jeweiligen Träger erfaßt und einen dem Stellungsfühler 29 nachgeschalteten Signalwandler 30. Dem Signalwandler 30 des Beleuchtungssystems 2 folgt unmittelbar die zugehörige Anzeige 24, während im Beobachtungssytem 3 zwischen den Signalwandler 30 und die Anzeige 24 ein Rechner 31 geschaltet ist. Zwei weitere Eingänge des Rechners 31 sind an die Dateneingabe 26 bzw. die Befehlseingabe 27 angeschlossen. Ein Ausgang des Rechners 31 wirkt auf einen in den Figuren 1 und 2 nicht gezeigten Antrieb 32 für den Träger des beweglichen Gliedes der Abbildungsoptik 9.

Bei dem in Fig. 6 gezeigten Handophthalmoskop lassen sich die Träger (Blendenträger 7, Filterträger 8, Linsenräder 13, 14) wie üblich mit Hilfe der Betätigungselemente 21, 22, 23 einstellen. Eine Rück- oder Nullstellung der einzelnen Träger ist mit Hilfe des Nustellungs-Betätigungselements 23 möglich. Das Erreichen der neutralen oder Null-Stellung wird mittels einer an den Signalwandler 30 des Beobachtungssystems 3 angeschlossenen akustischen und/oder optischen Anzeige signalisiert. Mit Hilfe der Dateneingabe 26 und der Befehlseingabe 27 können beliebige Daten und Befehle in den Rechner 31 eingegeben sowie im Rechner gespeicherte Programme aufgerufen werden. Dadurch ist es möglich, aus der Stellung der Linsenräder 13 und 14 unter Berücksichtigung individueller Untersuchungsparameter, z.B.

des Abstandes zwischen Ophthalmoskop und Patientenauge, der Untersucher-Refraktion, der bevorzugten Stellung der Funktionselemente und dergleichen, die wahre Patientenrefraktion oder auch Niveauunterschiede auf dem Augenhintergrund im Rechner 31 zu berechnen. Mit Hilfe des Antriebs 32 ist es möglich, das bewegliche Glied der Abbildungsoptik 9 entsprechend der ermittelten wahren Refraktion des Patienten nachzuführen, so daß sich die eingebauten Blenden oder Meßmarken auf dem Fundus des zu untersuchenden Auges scharf abbilden lassen. Dadurch kann zum Beispiel der mit der Refrakton des zu untersuchenden Auges variierende Abbildungsmaßstab von Meßmarken oder direkt die wahre Größe von Teilstrich-Abständen auf dem Fundus bei Bedarf angezeigt werden. Auch kann das oder gegebenenfalls mehrere bewegliche Glieder der Abbildungsoptik 9 so entlang ihrer optischen Achse verschoben werden, daß sich ein konstanter Abbildungsmaßstab auf dem Fundus ergibt.

HANDOPHTHALMOSKOP

Patentansprüche:

1. Handophthalmoskop, in dessen Beleuchtungssystem (2) eine längs
ihrer optischen Achse verschiebbare Abbildungsoptik (9) vorgesehen ist und/oder Blenden, Test- und Meßmarken eines Blendenträgers (7) und/oder die Filter eines Filterträgers (8) einschaltbar sind, und in dessen Beobachtungsstrahlengang die Linsen (15, 16) wenigstens eines Linsenträgers (13, 14) einschaltbar sind, und mit von der Außenseite des Gehäuses (1) zugänglichen bzw. sichtbaren Betätigungs- bzw. Anzeigeelementen (21,
22, 23, 24), dadurch g e k e n n z e i c h n e t , daß innerhalb des Gehäuses (1) für die Abbildungsoptik (9) und/oder
den Blendenträger (7) und/oder den Filterträger (8) und/oder
den Linsenträger (13, 14) je ein mit dem jeweiligen Träger gekoppelter elektromagnetischer oder elektromotorischer Antrieb
(17, 18, 19, 20, 32) angeordnet ist, daß die Betätigungselemente (21, 22, 23) an der Außenseite des Gehäuses (1) angeordnet sind, ohne die Gehäusewandung zu durchbrechen, und daß
den Trägern (7, 8, 13, 14) Stellungsfühler (29) zugeordnet sind,
die über Signalwandler (30) an die zugehörigen Anzeigeelemente
(24) angeschlossen ·sind.

2. Handophthalmoskop nach Anspruch 1, dadurch g e k e n n -
z e i c h n e t , daß zwischen die Betätigungselemente (21,
22, 23) und die Antriebe (17, 18, 19, 20, 32) eine elektronische Steuereinheit (28) geschaltet ist.

3. Handophthalmoskop nach Anspruch 1, dadurch g e k e n n -
z e i c h n e t , daß der/die Stellungsfühler (29) des Beobachtungssystems (3) an einen Rechner (31) angeschlossen sind.

4. Handophthalmoskop nach Anspruch 3, g e k e n n z e i c h - n e t durch eine Daten- und eine Befehlseingabe (26, 27) für den Rechner (31), die auf der Außenseite des Gehäuses (1) angeordnet sind.

5. Handophthalmoskop nach Anspruch 3, dadurch g e k e n n - z e i c h n e t , daß ein Ausgang des Rechners (31) an den Antrieb (32) der Abbildungsoptik (9) angeschlossen ist.

6. Handophthalmoskop nach einem der vorstehenden Ansprüche, g e k e n n z e i c h n e t durch einen auf der Außenseite des Gehäuses (1) angeordneten und an die Steuereinheit (28) angeschlossenen Nullstellungs-Befehlsgeber (23).

7. Handophthalmoskop nach Anspruch 6, g e k e n n z e i c h - n e t durch eine an den/die Signalwandler (30) zumindest des Beobachtungssystems (3) angeschlossene optische und/oder akustische Anzeige (33).

Fig. 1

EPA-30334

**0080623**

Fig. 2

Fig. 5

Fig. 4

Fig. 3

Fig. 6